# EUROPÄISCHE PATENTSCHRIFT

(11) **EP 3 135 641 B1**
(45) Veröffentlichungstag und Bekanntmachung des Hinweises auf die Patenterteilung: **03.07.2024**
(21) Anmeldenummer: 15190547.8
(22) Anmeldetag: 20.10.2015
(51) Int. Cl.: C03C 3/087, C03C 3/097, C03C 10/00

(54) **LITHIUMSILIKAT-TIEFQUARZ-GLASKERAMIK**
LITHIUM SILICATE GLASS CERAMIC WITH ALPHA QUARTZ
VITROCÉRAMIQUE EN ALPHA QUARTZ ET SILICATE DE LITHIUM

(30) Priorität: 25.08.2015 EP 15182307
(43) Veröffentlichungstag der Anmeldung: 01.03.2017
(60) Teilanmeldung: 23198357.8
(73) Patentinhaber: Ivoclar Vivadent AG, 9494 Schaan (LI)
(72) Erfinder: Ritzberger, Christian, 9472 Grabs (CH); Rampf, Markus, 8853 Lachen (CH); Höland, Wolfram, 9494 Schaan (LI); Dittmer, Marc, 6800 Feldkirch (AT); Schweiger, Marcel, 7000 Chur (CH)
(74) Vertreter: Uexküll & Stolberg

(56) Entgegenhaltungen:
- EP-A1- 3 050 856

## Beschreibung

Die Erfindung betrifft Lithiumsilikat-Tiefquarz-Glaskeramik, die sich zum Einsatz in der Zahnheilkunde, bevorzugt zur Herstellung von dentalen Restaurationen, eignet sowie Vorstufen zur Herstellung dieser Glaskeramik.

Lithiumsilikat-Glaskeramiken zeichnen sich in der Regel durch sehr gute mechanische Eigenschaften aus, weshalb sie seit einiger Zeit im Dentalbereich und dort vornehmlich zur Herstellung von Dentalkronen und kleinen Dentalbrücken Anwendung finden.

Die US 5,507,981 und US 5,702,514 beschreiben Lithiumdisilikat-Glaskeramiken, die durch Verpressen im viskosen Zustand zu Dentalrestaurationen verarbeitet werden. Dabei ist allerdings die Verwendung eines deformierbaren Tiegels zwingend erforderlich, was die Verarbeitung sehr aufwändig gestaltet.

Die EP 827 941 und EP 916 625 offenbaren Lithiumdisilikat-Glaskeramiken, denen durch Verpressen oder maschinelle Bearbeitung die Form der gewünschten Dentalrestauration gegeben werden kann.

Die EP 1 505 041 und die EP 1 688 398 beschreiben Verfahren zur Herstellung von Dentalrestaurationen aus Lithiumdisilikat-Glaskeramiken. Dabei wird zunächst als Zwischenstufe eine Glaskeramik mit Lithiummetasilikat als Hauptkristallphase erzeugt, die sich sehr gut mechanisch z.B. mittels CAD/CAM-Verfahren, bearbeiten lässt. Diese Zwischenstufe wird dann einer weiteren Wärmebehandlung unterzogen, um die gewünschte hochfeste Lithiumdisilikat-Glaskeramik zu bilden. Die während des Verfahrens verwendeten Wärmebehandlungen sollen so gewählt sein, dass die Bildung von unerwünschten Kristallphasen, wie zum Beispiel von Cristobalit, vermieden werden.

Die WO 2013/053864 offenbart Lithiumsilikat-Glaskeramiken, die zweiwertiges Metalloxid enthalten und durch Heißpressen sowie durch maschinelle Bearbeitung zu Dentalrestaurationen verarbeitet werden können.

Aus der WO 2013/164256 sind Glaskeramiken bekannt, die Lithiumdisilikat als Hauptkristallphase und Apatit als eine weitere Kristallphase aufweisen. Die Glaskeramiken zeichnen sich durch hohe chemische Stabilität aus und können durch maschinelle Bearbeitung oder Heißpressen zu den gewünschten Dentalrestaurationen verformt werden.

Die US 2015/0104655 beschreibt Glaskeramiken, die in Abhängigkeit von der Zusammensetzung und der für die Kristallisation gewählten Temperaturbehandlung Lithiumdisilikat, Lithiummetasilikat, Lithiumphosphat, Cristobalit, Tridymit, Quarz oder Spodumen als Kristallphasen enthalten können. Die Glaskeramiken sind insbesondere zur Verblendung von Zirkoniumoxid-Keramiken vorgesehen.

Die maschinelle Bearbeitung der konventionellen Lithiumdisilikat-Glaskeramiken ist aufgrund ihrer hohen Festigkeit jedoch nur schwierig möglich und sie geht daher regelmäßig mit einem hohen Verschleiß der eingesetzten Werkzeuge einher. Die ebenfalls mögliche maschinelle Bearbeitung entsprechender Lithiummetasilikat-Glaskeramiken als Vorstufen ist deutlich einfacher. Sie erfordert aber nach der Formgebung durch maschinelle Bearbeitung noch eine weitere Wärmebehandlung zur Erzeugung der Restauration aus hochfester Lithiumdisilikat-Glaskeramik.

Es besteht daher ein Bedarf an Lithiumsilikat-Glaskeramiken, die einfach maschinell bearbeitbar sind und nach dieser Bearbeitung keiner weiteren Wärmebehandlung bedürfen, um der erzeugten Dentalrestauration die gewünschten mechanischen Eigenschaften zu verleihen. Dabei sollen die Lithiumsilikat-Glaskeramiken nicht nur über sehr gute mechanische Eigenschaften, sondern gleichfalls auch über sehr gute optische Eigenschaften verfügen, damit sie auch den hohen ästhetischen Ansprüchen gerecht werden, die an ein restauratives Dentalmaterial gestellt werden.

Diese Aufgabe wird durch die Verwendung nach den Ansprüchen 1 bis 20 gelöst.

Gegenstand der Erfindung ist ebenfalls das Verfahren nach dem Anspruch 21.

Die erfindungsgemäße Lithiumsilikat-Tiefquarz-Glaskeramik zeichnet sich dadurch aus, dass sie Lithiumsilikat als Hauptkristallphase und Tiefquarz als weitere Kristallphase enthält.

Es hat sich überraschenderweise gezeigt, dass die erfindungsgemäße Glaskeramik eine Kombination von sehr wünschenswerten mechanischen und optischen Eigenschaften in sich vereinigt, wie sie gerade für ein restauratives Dentalmaterial erforderlich sind. Die Glaskeramik hat eine hohe Festigkeit und dennoch kann ihr in einfacher Weise durch maschinelle Bearbeitung die Form einer Dentalrestauration gegeben werden. Eine nachträgliche Wärmebehandlung zur Erzielung einer zufriedenstellenden Festigkeit ist nicht erforderlich. Es war weiterhin nicht zu erwarten, dass durch das Vorsehen von Tiefquarz als weiterer Kristallphase neben Lithiumsilikat als Hauptkristallphase dennoch sehr gute optische Eigenschaften erzielt werden können. Denn viele Nebenkristallphasen haben einen negativen Effekt auf die optischen Eigenschaften von Lithiumsilikat-Glaskeramiken. Sie können beispielsweise die Transluzenz vermindern und sie können ebenfalls die Einfärbbarkeit der Glaskeramik beeinträchtigen, was bei der Nachahmung der Farbe des zu ersetzenden natürlichen Zahnmaterials zu erheblichen Schwierigkeiten führen kann.

Die erfindungsgemäße Lithiumsilikat-Tiefquarz-Glaskeramik enthält insbesondere 59,0 bis 79,0, bevorzugt 64,0 bis 78,0 und besonders bevorzugt 64,0 bis 76,0 Gew.-% SiO₂.

In einer anderen Ausführungsform enthält die erfindungsgemäße Lithiumsilikat-Tiefquarz-Glaskeramik insbesondere 68,0 bis 79,0, bevorzugt 69,0 bis 78,0 und besonders bevorzugt 70,0 bis 76,0 Gew.-% SiO₂.

Es ist weiter bevorzugt, dass die erfindungsgemäße Lithiumsilikat-Tiefquarz-Glaskeramik 8,0 bis 15,0, besonders bevorzugt 9,0 bis 14,0 und ganz besonders bevorzugt 10,0 bis 13,5 Gew.% Li₂O enthält. Es wird angenommen, dass Li₂O die Viskosität der Glasmatrix erniedrigt und damit die Kristallisation der gewünschten Phasen fördert.

In einer weiteren bevorzugten Ausführungsform enthält die Glaskeramik 0 bis 9,0, bevorzugt 2,0 bis 6,0 und besonders bevorzugt 3,0 bis 5,0 Gew.-% P₂O₅. Es wird angenommen, dass das P₂O₅ als Keimbildner wirkt.

Die Glaskeramik enthält 1,0 bis 8,0 und insbesondere 2,0 bis 7,0 Gew.-% Oxid einwertiger Elemente Me^{I}₂O ausgewählt aus der Gruppe von K₂O, Na₂O, Rb₂O, Cs₂O und Mischungen davon enthält.

Besonders bevorzugt enthält die Glaskeramik mindestens eines und insbesondere alle der folgenden Oxide einwertiger Elemente Me^{I}₂O in den angegebenen Mengen:

| Komponente | Gew.-% |
|---|---|
| K₂O | 0 bis 5,0 |
| Na₂O | 0 bis 2,0 |
| Rb₂O | 0 bis 8,0 |
| Cs₂O | 0 bis 7,0. |

In einer besonders bevorzugten Ausführungsform enthält die erfindungsgemäße Glaskeramik 0 bis 5,0, bevorzugt 1,0 bis 4,0 und besonders bevorzugt 2,0 bis 3,5 Gew.-% K₂O.

Des Weiteren ist es bevorzugt, dass die Glaskeramik 1,0 bis 9,0, bevorzugt 2,0 bis 8,0 und besonders bevorzugt 3,0 bis 7,0 Gew.-% Oxid zweiwertiger Elemente Me^{II}O ausgewählt aus der Gruppe von CaO, MgO, SrO, ZnO und Mischungen davon enthält.

In einer weiteren bevorzugten Ausführungsform enthält die Glaskeramik weniger als 2,0 Gew.-% an BaO. Die Glaskeramik ist insbesondere im Wesentlichen frei von BaO.

Bvorzugt enthält die Glaskeramik mindestens eines und insbesondere alle der folgenden Oxide zweiwertiger Elemente Me^{II}O in den angegebenen Mengen:

| Komponente | Gew.-% |
|---|---|
| CaO | 0 bis 3,0 |
| MgO | 0 bis 6,0 |
| SrO | 0 bis 4,0 |
| ZnO | 0 bis 9,0 |

Die erfindungsgemäße Glaskeramik enthält 1,0 bis 6,0, insbesondere 1,5 bis 6,0, bevorzugt 2,0 bis 5,5, besonders bevorzugt 3,1 bis 5,5 und ganz besonders bevorzugt 3,4 bis 5,0 Gew.-% MgO.

Es ist weiter eine Glaskeramik bevorzugt, die 0 bis 8,0, bevorzugt 1,0 bis 7,0 und besonders bevorzugt 2,0 bis 6,5 Gew.-% Oxid dreiwertiger Elemente Me^{III}₂O₃ ausgewählt aus der Gruppe von Al₂O₃, B₂O₃, Y₂O₃, La₂O₃, Ga₂O₃, In₂O₃ und Mischungen davon enthält.

Besonders bevorzugt enthält die Glaskeramik mindestens eines und insbesondere alle der folgenden Oxide dreiwertiger Elemente Me^{III}₂O₃ in den angegebenen Mengen:

| Komponente | Gew.-% |
|---|---|
| Al₂O₃ | 1,0 bis 6,0 |
| B₂O₃ | 0 bis 4,0 |
| Y₂O₃ | 0 bis 5,0 |
| La₂O₃ | 0 bis 5,0 |
| Ga₂O₃ | 0 bis 3,0 |
| In₂O₃ | 0 bis 5,0 |

In einer besonders bevorzugten Ausführungsform enthält die erfindungsgemäße Glaskeramik 1,0 bis 6,0 und bevorzugt 2,0 bis 5,0 Gew.-% Al₂O₃.

Ferner ist eine Glaskeramik bevorzugt, die 0 bis 10,0 und besonders bevorzugt 0 bis 8,0 Gew.-% Oxid vierwertiger Elemente Me^{IV}O₂ ausgewählt aus der Gruppe von ZrO₂, TiO₂, SnO₂, CeO₂, GeO₂ und Mischungen davon enthält.

Besonders bevorzugt enthält die Glaskeramik mindestens eines und insbesondere alle der folgenden Oxide vierwertiger Elemente Me^{IV}O₂ in den angegebenen Mengen:

| Komponente | Gew.-% |
|---|---|
| ZrO₂ | 0 bis 3,0 |
| TiO₂ | 0 bis 4,0 |
| SnO₂ | 0 bis 3,0 |
| GeO₂ | 0 bis 9,0, insbesondere 0 bis 8,0 |
| CeO₂ | 0 bis 4,0. |

In einer weiteren Ausführungsform enthält die Glaskeramik 0 bis 8,0, bevorzugt 0 bis 6,0 Gew.-% Oxid fünfwertiger Elemente Me^{V}₂O₅ ausgewählt aus der Gruppe von aus V₂O₅, Ta₂O₅, Nb₂O₅ und Mischungen davon.

Besonders bevorzugt enthält die Glaskeramik mindestens eines und insbesondere alle der folgenden Oxide fünfwertiger Elemente Me^{V}₂O₅ in den angegebenen Mengen:

| Komponente | Gew.-% |
|---|---|
| V₂O₅ | 0 bis 2,0 |
| Ta₂O₅ | 0 bis 5,0 |
| Nb₂O₅ | 0 bis 5,0 |

In einer weiteren Ausführungsform enthält die Glaskeramik 0 bis 5,0, bevorzugt 0 bis 4,0 Gew.-% Oxid sechswertiger Element Me^{VI}O₃ ausgewählt aus der Gruppe von WO₃, MoO₃ und Mischungen davon.

Besonders bevorzugt enthält die Glaskeramik mindestens eines und insbesondere alle der folgenden Oxide Me^{VI}O₃ in den angegebenen Mengen:

| Komponente | Gew.-% |
|---|---|
| WO₃ | 0 bis 3,0 |
| MoO₃ | 0 bis 3,0 |

In einer weiteren Ausführungsform enthält die erfindungsgemäße Glaskeramik 0 bis 1,0 und insbesondere 0 bis 0,5 Gew.-% Fluor.

Besonders bevorzugt ist eine Glaskeramik, die mindestens eine und bevorzugt alle der folgenden Komponenten in den angegebenen Mengen enthält:

| Komponente | Gew.-% |
|---|---|
| SiO₂ | 59,0 bis 79,0 oder 68,0 bis 79,0 |
| Li₂O | 8,0 bis 15,0 |
| P₂O₅ | 0 bis 9,0 |
| Me^{I}₂O | 1,0 bis 8,0 |
| Me^{II}O | 1,0 bis 9,0 |
| Me^{III}₂O₃ | 0 bis 8,0 |
| Me^{IV}O₂ | 0 bis 10,0 |
| Me^{V}₂O₅ | 0 bis 8,0 |
| Me^{VI}O₃ | 0 bis 5,0 |
| Fluor | 0 bis 1,0, |

wobei Me^{I}₂O, Me^{II}O, Me^{III}₂O₃, Me^{IV}O₂, Me^{V}₂O₅ und Me^{VI}O₃ die oben angegebene Bedeutung haben.

In einer weiteren besonders bevorzugten Ausführungsform enthält die Glaskeramik mindestens eine und bevorzugt alle der folgenden Komponenten in den angegebenen Mengen:

| Komponente | Gew.-% |
|---|---|
| SiO₂ | 59,0 bis 79,0 oder 68,0 bis 79,0 |
| Li₂O | 8,0 bis 15,0 |
| P₂O₅ | 0 bis 9,0 |
| K₂O | 0 bis 5,0 |
| Na₂O | 0 bis 2,0 |
| Rb₂O | 0 bis 8,0 |
| Cs₂O | 0 bis 7,0 |
| CaO | 0 bis 3,0 |
| MgO | 0 bis 6,0 |
| SrO | 0 bis 4,0 |
| ZnO | 0 bis 9,0 |
| Al₂O₃ | 1, 0 bis 6,0 |
| B₂O₃ | 0 bis 4,0 |
| Y₂O₃ | 0 bis 5,0 |
| La₂O₃ | 0 bis 5,0 |
| Ga₂O₃ | 0 bis 3,0 |
| In₂O₃ | 0 bis 5,0 |
| ZrO₂ | 0 bis 3,0 |
| TiO₂ | 0 bis 4,0 |
| SnO₂ | 0 bis 3,0 |
| GeO₂ | 0 bis 9,0, insbesondere 0 bis 8,0 |
| CeO₂ | 0 bis 4,0 |
| V₂O₅ | 0 bis 2,0 |
| Ta₂O₅ | 0 bis 5,0 |
| Nb₂O₅ | 0 bis 5,0 |
| WO₃ | 0 bis 3,0 |
| MoO₃ | 0 bis 3,0 |
| Fluor | 0 bis 1,0. |

Manche der vorstehend genannten Komponenten können als Färbemittel und/oder Fluoreszenzmittel dienen. Die erfindungsgemäße Glaskeramik kann darüber hinaus noch weitere Färbemittel und/oder Fluoreszenzmittel enthalten. Diese können z.B. aus Bi₂O₃ oder Bi₂O₅ und insbesondere aus weiteren anorganischen Pigmenten und/oder Oxiden von d- und f-Elementen, wie z.B. den Oxiden von Mn, Fe, Co, Pr, Nd, Tb, Er, Dy, Eu und Yb, ausgewählt sein. Mithilfe dieser Färbemittel und Fluoreszenzmittel ist eine einfache Einfärbung der Glaskeramik möglich, um die gewünschten optischen Eigenschaften insbesondere von natürlichem Zahnmaterial zu imitieren. Es ist überraschend, dass dies trotz des als weiterer Kristallphase vorhandenen Tiefquarzes problemlos möglich ist.

In einer bevorzugten Ausführungsform der Glaskeramik liegt das Molverhältnis von SiO₂ zu Li₂O im Bereich von 2,2 bis 4,1, bevorzugt 2,2 bis 3,8 und besonders bevorzugt 2,2 bis 3,5. Es ist überraschend, dass innerhalb dieser breiten Bereiche die Herstellung der erfindungsgemäßen Glaskeramik mit Lithiumsilikat als Hauptkristallphase und Tiefquarz als weiterer Kristallphase gelingt.

Mit dem Begriff "Hauptkristallphase" wird die Kristallphase bezeichnet, die von allen in der Glaskeramik vorhandenen Kristallphasen den höchsten Massenanteil hat. Die Bestimmung der Massen der Kristallphasen erfolgt dabei insbesondere mit der Rietveld-Methode. Ein geeignetes Verfahren zur quantitativen Analyse der Kristallphasen mittels der Rietveld-Methode ist z.B. in der Dissertation von M. Dittmer "Gläser und Glaskeramiken im System MgO-Al2O3-SiO2 mit ZrO2 als Keimbildner", Universität Jena 2011, beschrieben.

Es ist bevorzugt, dass die erfindungsgemäße Glaskeramik Lithiumdisilikat oder Lithiummetasilikat als Hauptkristallphase enthält. In einer besonders bevorzugten Ausführungsform enthält die erfindungsgemäße Glaskeramik Lithiumdisilikat als Hauptkristallphase, da diese Glaskeramik über eine besonders vorteilhafte Kombination an wünschenswerten Eigenschaften verfügt.

Bei einer erfindungsgemäßen Glaskeramik mit Lithiummetasilikat als Hauptkristallphasen ist es bevorzugt, dass die Glaskeramik auch Lithiumdisilikat als weitere Kristallphase neben Tiefquarz enthält.

Es ist bevorzugt, dass die erfindungsgemäße Glaskeramik mindestens 20 Gew.-%, bevorzugt 25 bis 55 Gew.-% und besonders bevorzugt 30 bis 55 Gew.% Lithiumdisilikat-Kristalle aufweist.

Es ist weiter bevorzugt, dass die erfindungsgemäße Glaskeramik 0,2 bis 28 Gew.-% und besonders bevorzugt 0,5 bis 25 Gew.-% Tiefquarz-Kristalle aufweist.

Die erfindungsgemäße Glaskeramik kann zusätzlich zu Lithiumsilikat und Tiefquarz noch weitere Kristallphasen, wie z.B. Apatit, Cäsiumalumosilikat und insbesondere Lithiumphosphat enthalten. Dabei sollte die Menge an Cristobalit jedoch möglichst klein sein und insbesondere weniger als 1,0 Gew.-% betragen. Es ist besonders bevorzugt, dass die erfindungsgemäße Glaskeramik im Wesentlichen frei von Cristobalit ist.

Die Art und insbesondere die Menge der gebildeten Kristallphasen können durch die Zusammensetzung des Ausgangsglases sowie die Wärmebehandlung gesteuert werden, die zur Herstellung der Glaskeramik aus dem Ausgangsglas angewendet wird. Die Beispiele veranschaulichen dies anhand der Variation der Zusammensetzung des Ausgangsglases und der angewendeten Wärmebehandlung.

Die Glaskeramik weist eine hohe biaxiale Bruchfestigkeit von vorzugsweise mindestens 200 MPa und besonders bevorzugt 250 bis 460 MPa auf. Die biaxiale Bruchfestigkeit wurde gemäß ISO 6872 (2008) (Kolben-auf-drei-Kugeln-Prüfung) bestimmt.

Es ist besonders überraschend, dass die erfindungsgemäße Glaskeramik trotz dieser hohen Bruchfestigkeit einfach und schnell maschinell mittels computergestützter Fräs- und Schleifgeräte bearbeitet werden kann, um die Glaskeramik z.B. in die Form einer Dentalrestauration zu bringen.

Die erfindungsgemäße Glaskeramik weist einen thermischen Ausdehnungskoeffizienten WAK (gemessen im Bereich von 100 bis 500°C) von bevorzugt 9,5 bis 14,0·10⁻⁶ K⁻¹ auf. Der WAK wird gemäß ISO 6872 (2008) bestimmt. Eine Einstellung des Wärmeausdehnungskoeffizienten auf einen gewünschten Wert erfolgt insbesondere durch die Art und Menge der in der Glaskeramik vorhandenen Kristallphasen sowie die chemische Zusammensetzung der Glaskeramik.

Die Transluzenz der Glaskeramik wurde in Form des Kontrastwerts (CR-Wert) gemäß British Standard BS 5612 bestimmt, und dieser Kontrastwert betrug vorzugsweise 40 bis 92.

Die bei der erfindungsgemäßen Glaskeramik vorliegende besondere Kombination an Eigenschaften erlaubt es sogar, sie als Dentalmaterial und insbesondere als Material zur Herstellung von Dentalrestaurationen einzusetzen.

Die Erfindung betrifft ebenfalls verschiedene Vorstufen mit entsprechender Zusammensetzung, aus denen die erfindungsgemäße Lithiumsilikat-Tiefquarz-Glaskeramik durch Wärmebehandlung hergestellt werden kann. Diese Vorstufen sind ein entsprechend zusammengesetztes Ausgangsglas und ein entsprechend zusammengesetztes Ausgangsglas mit Keimen. Die Bezeichnung "entsprechende Zusammensetzung" bedeutet, dass diese Vorstufen die gleichen Komponenten in den gleichen Mengen wie die Glaskeramik enthalten, wobei die Komponenten mit Ausnahme von Fluor als Oxide berechnet werden, so wie es bei Gläsern und Glaskeramiken üblich ist.

Die Erfindung betrifft daher ebenfalls ein Ausgangsglas, das die Komponenten der erfindungsgemäßen Lithiumdisilikat-Tiefquarz-Glaskeramik enthält.

Das erfindungsgemäße Ausgangsglas enthält daher insbesondere geeignete Mengen an SiO₂ und Li₂O, die zur Ausbildung der erfindungsgemäßen Glaskeramik mit Lithiumsilikat als Hauptkristallphase und Tiefquarz als weiterer Kristallphase erforderlich sind. Weiter kann das Ausgangsglas auch noch andere Komponenten enthalten, wie sie oben für die erfindungsgemäße Lithiumsilikat-Tiefquarz-Glaskeramik angegeben sind. Es sind alle solche Ausführungsformen für die Komponenten des Ausgangsglases bevorzugt, die auch für die Komponenten der erfindungsgemäßen Lithiumsilikat-Tiefquarz-Glaskeramik als bevorzugt angegeben sind.

Die Erfindung betrifft ebenfalls ein solches Ausgangsglas, das Keime für die Kristallisation von Lithiummetasilikat, Lithiumdisilikat und/oder Tiefquarz enthält.

Durch Wärmebehandlung des Ausgangsglases kann zunächst die weitere Vorstufe Ausgangsglas mit Keimen erzeugt werden. Durch Wärmebehandlung dieser weiteren Vorstufe kann dann die erfindungsgemäße Lithiumsilikat-Tiefquarz-Glaskeramik erzeugt werden. Es ist bevorzugt, die erfindungsgemäße Lithiumsilikat-Tiefquarz-Glaskeramik durch Wärmebehandlung des Ausgangsglases mit Keimen zu bilden.

Es ist bevorzugt, das Ausgangsglas einer Wärmebehandlung bei einer Temperatur von 400 bis 600°C, insbesondere 450 bis 550°C, für eine Dauer von bevorzugt 5 bis 120 min, insbesondere 10 bis 60 min, zu unterwerfen, um das Ausgangsglas mit Keimen für die Kristallisation von Lithiummetasilikat, Lithiumdisilikat und/oder Tiefquarz zu erzeugen.

Es ist weiter bevorzugt, das Ausgangsglas mit Keimen einer Wärmebehandlung bei einer Temperatur von 700 bis 900°C für eine Dauer von insbesondere 1 bis 120 min, bevorzugt 5 bis 120 min, besonders bevorzugt 10 bis 60 min, zu unterwerfen, um die Lithiumsilikat-Tiefquarz-Glaskeramik herzustellen. Zur Herstellung der Lithiumsilikat-Tiefquarz-Glaskeramik erfolgt die Wärmebehandlung des Ausgangsglases mit Keimen besonders bevorzugt bei 700 bis 880°C, insbesondere 750 bis 850°C, für eine Dauer von bevorzugt 5 bis 120 min, besonders bevorzugt 10 bis 60 min.

Die Erfindung betrifft ebenfalls ein Verfahren zur Herstellung der erfindungsgemäßen Lithiumsilikat-Tiefquarz-Glaskeramik, bei dem das Ausgangsglas oder das Ausgangsglas mit Keimen mindestens einer Wärmebehandlung bei einer Temperatur von 700 bis 900°C für eine Dauer von insbesondere 1 bis 120 min, bevorzugt 5 bis 120 min und besonders bevorzugt 10 bis 60 min, unterzogen wird.

Das Ausgangsglas und das Ausgangsglas mit Keimen kann z.B. in Form eines Massivglasrohlings, eines Pulverpresslings oder eines Pulvers der mindestens einen Wärmebehandlung unterzogen werden. Die im erfindungsgemäßen Verfahren durchgeführte mindestens eine Wärmebehandlung kann auch im Rahmen eines Heißpressens oder Aufsinterns des erfindungsgemäßen Ausgangsglases oder des erfindungsgemäßen Ausgangsglases mit Keimen erfolgen.

Das erfindungsgemäße Vefahren umfasst
(a) die Wärmebehandlung des Ausgangsglases bei einer Temperatur von 400 bis 600°C, um das Ausgangsglas mit Keimen zu bilden, und
(b) die Wärmebehandlung des Ausgangsglases mit Keimen bei einer Temperatur von 700 bis 900°C, um die Lithiumsilikat-Tiefquarz-Glaskeramik zu bilden.

Die Dauer der bei (a) und (b) durchgeführten Wärmebehandlungen beträgt 5 bis 120 min und bevorzugt 10 bis 60 min.

Zur Herstellung des Ausgangsglases wird insbesondere so vorgegangen, dass eine Mischung von geeigneten Ausgangsmaterialien, wie z.B. Carbonaten, Oxiden, Phosphaten und Fluoriden, bei Temperaturen von insbesondere 1300 bis 1600°C für 2 bis 10 h erschmolzen wird. Zur Erzielung einer besonders hohen Homogenität wird die erhaltene Glasschmelze in Wasser gegossen, um ein Glasgranulat zu bilden, und das erhaltene Granulat wird dann erneut aufgeschmolzen.

Die Schmelze kann dann in Formen gegossen werden, um Rohlinge des Ausgangsglases, sogenannte Massivglasrohlinge oder monolithische Rohlinge, zu erzeugen.

Es ist ebenfalls möglich, die Schmelze erneut in Wasser zu geben, um ein Granulat herzustellen. Dieses Granulat kann nach Mahlen und gegebenenfalls Zugabe weiterer Komponenten, wie Färbe- und Fluoreszenzmitteln, zu einem Rohling, einem sogenannten Pulverpressling, gepresst werden.

Schließlich kann das Ausgangsglas nach Granulierung auch zu einem Pulver verarbeitet werden.

Anschließend wird das Ausgangsglas, z.B. in Form eines Massivglasrohlings, eines Pulverpresslings oder in Form eines Pulvers, mindestens einer Wärmebehandlung unterzogen. Es ist bevorzugt, dass zunächst eine erste Wärmebehandlung durchgeführt wird, um ein erfindungsgemäßes Ausgangsglas mit Keimen herzustellen, welche zur Bildung von Lithiummetasilikat-, Lithiumdisilikat- und/oder Tiefquarz-Kristallen geeignet sind. Das Glas mit Keimen wird dann üblicherweise mindestens einer weiteren Temperaturbehandlung bei einer höheren Temperatur unterworfen, um Kristallisation von Lithiumsilikat, insbesondere von Lithiumdisilikat, und Tiefquarz zu bewirken.

Die erfindungsgemäßen Glaskeramiken und die erfindungsgemäßen Gläser liegen insbesondere in Form von Pulvern, Granulaten oder Rohlingen in beliebiger Form und Größe, z.B. monolithischen Rohlingen, wie Plättchen, Quadern oder Zylindern, oder Pulverpresslingen, in ungesinterter, teilgesinterter oder dichtgesinterter Form, vor. In diesen Formen können sie einfach weiterverarbeitet werden. Sie können aber auch in Form von dentalen Restaurationen, wie Inlays, Onlays, Kronen, Veneers, Schalen oder Abutments, vorliegen.

Aus den erfindungsgemäßen Glaskeramiken und den erfindungsgemäßen Gläsern können dentale Restaurationen, wie Brücken, Inlays, Onlays, Kronen, Veneers, Schalen oder Abutments, hergestellt werden. Die Erfindung betrifft daher auch deren Verwendung zur Herstellung dentaler Restaurationen. Dabei ist es bevorzugt, dass der Glaskeramik oder dem Glas durch Verpressen oder maschinelle Bearbeitung die Form der gewünschten dentalen Restauration gegeben wird.

Das Verpressen erfolgt üblicherweise unter erhöhtem Druck und erhöhter Temperatur. Es ist bevorzugt, dass das Verpressen bei einer Temperatur von 700 bis 1200°C erfolgt. Weiter ist es bevorzugt, das Verpressen bei einem Druck von 2 bis 10 bar durchzuführen. Beim Verpressen wird durch viskoses Fließen des eingesetzten Materials die gewünschte Formänderung erreicht. Es können für das Verpressen das erfindungsgemäße Ausgangsglas und insbesondere das erfindungsgemäße Ausgangsglas mit Keimen, und die erfindungsgemäße Lithiumsilikat-Tiefquarz-Glaskeramik verwendet werden. Dabei können die erfindungsgemäßen Gläser und Glaskeramiken insbesondere in Form von Rohlingen in beliebiger Form und Größe, z.B. Massivrohlingen oder Pulverpresslingen, z.B. in ungesinterter, teilgesinterter oder dichtgesinterter Form, eingesetzt werden.

Die maschinelle Bearbeitung erfolgt üblicherweise durch materialabtragende Verfahren und insbesondere durch Fräsen und/oder Schleifen. Es ist besonders bevorzugt, dass die maschinelle Bearbeitung im Rahmen eines CAD/CAM-Verfahrens durchgeführt wird. Für die maschinelle Bearbeitung können das erfindungsgemäße Ausgangsglas, das erfindungsgemäße Ausgangsglas mit Keimen und die erfindungsgemäße Lithiumsilikat-Tiefquarz-Glaskeramik verwendet werden. Dabei können die erfindungsgemäßen Gläser und Glaskeramiken insbesondere in Form von Rohlingen, z.B. Massivrohlingen oder Pulverpresslingen, z.B. in ungesinterter, teilgesinterter oder dichtgesinterter Form, eingesetzt werden. Für die maschinelle Bearbeitung wird bevorzugt die erfindungsgemäße Lithiumsilikat-Tiefquarz-Glaskeramik verwendet.

Nach der Herstellung der gewünscht geformten dentalen Restauration, z.B. durch Verpressen oder maschinelle Bearbeitung, kann diese noch wärmebehandelt werden, um die Porosität, z.B. eines eingesetzten porösen Pulverpresslings, zu vermindern.

Die erfindungsgemäßen Glaskeramiken und die erfindungsgemäßen Gläser eignen sich allerdings auch als Beschichtungsmaterial von z.B. Keramiken und Glaskeramiken. Die Erfindung ist daher ebenfalls auf die Verwendung der erfindungsgemäßen Gläser oder der erfindungsgemäßen Glaskeramiken zur Beschichtung von insbesondere Keramiken und Glaskeramiken gerichtet.

Die Erfindung betrifft auch ein Verfahren zur Beschichtung von Keramiken, Metallen, Metalllegierungen und Glaskeramiken, bei dem erfindungsgemäße Glaskeramik oder erfindungsgemäßes Glas auf die Keramik oder Glaskeramik aufgebracht und erhöhter Temperatur ausgesetzt wird.

Dies kann insbesondere durch Aufsintern oder durch Fügen eines mittels CAD-CAM hergestellten Überwurfs mit einem geeigneten Glaslot oder Kleber und bevorzugt durch Aufpressen erfolgen. Beim Aufsintern wird die Glaskeramik oder das Glas in üblicher Weise, z.B. als Pulver, auf das zu beschichtende Material, wie Keramik oder Glaskeramik, aufgebracht und anschließend bei erhöhter Temperatur gesintert. Bei dem bevorzugten Aufpressen wird erfindungsgemäße Glaskeramik oder erfindungsgemäßes Glas, z.B. in Form von Pulverpresslingen oder monolithischen Rohlingen, bei einer erhöhten Temperatur, von z.B. 700 bis 1200°C, und unter Anwendung von Druck, z.B. 2 bis 10 bar, aufgepresst. Hierzu können insbesondere die in der EP 231 773 beschriebenen Verfahren und der dort offenbarte Pressofen eingesetzt werden. Ein geeigneter Ofen ist z.B. der Programat EP 5000 von Ivoclar Vivadent AG, Liechtenstein.

Es ist bevorzugt, dass nach Abschluss des Beschichtungsvorganges die erfindungsgemäße Glaskeramik mit Lithiumsilikat, insbesondere Lithiumdisilikat, als Hauptkristallphase und Tiefquarz als weiterer Kristallphase vorliegt, da eine solche Glaskeramik über besonders gute Eigenschaften verfügt.

Aufgrund der vorstehend geschilderten Eigenschaften der erfindungsgemäßen Glaskeramiken und der erfindungsgemäßen Gläser eignen sich diese insbesondere zum Einsatz in der Zahnheilkunde. Gegenstand der Erfindung ist daher auch die Verwendung der erfindungsgemäßen Glaskeramiken oder der erfindungsgemäßen Gläser als Dentalmaterial und insbesondere zur Herstellung dentaler Restaurationen oder als Beschichtungsmaterial für dentale Restaurationen, wie Kronen, Brücken und Abutments.

Die Erfindung wird im Folgenden anhand von sie nichtbeschränkenden Beispielen näher erläutert.

### Beispiele

### Beispiele 1 bis 34 - Zusammensetzung und Kristallphasen

Es wurden insgesamt 34 Gläser und Glaskeramiken mit der aus Tabelle I angegebenen Zusammensetzung über Erschmelzung entsprechender Ausgangsgläser sowie anschließende Wärmebehandlungen zur gesteuerten Keimbildung und Kristallisation hergestellt.

Die angewendeten Wärmebehandlungen zur gesteuerten Keimbildung und gesteuerten Kristallisation sind ebenfalls in der Tabelle I angegeben. Dabei bedeuten
- T_{g}: Glasübergangstemperatur, bestimmt mittels DSC
- T_{S} und t_{S}: Angewendete Temperatur und Zeit für Erschmelzung des Ausgangsglases
- T_{Kb} und t_{Kb}: Angewendete Temperatur und Zeit für Keimbildung des Ausgangsglases
- T_{C} und t_{C}: Angewendete Temperatur und Zeit für die Kristallisation
- T_{Press} und t_{Press}: Angewendete Temperatur und Zeit für Kristallisation durch Heißpressen
- CR-Wert: Kontrastwerts der Glaskeramik gemäß British Standard BS 5612 bestimmt unter Verwendung von:
Gerät: Spektrometer CM-3700d (Konica-Minolta)
Messparameter:
Messfläche: 7mm x 5 mm
Messart: Remission / Reflektion
Messbereich: 400 nm -700 nm
Probengröße:
Durchmesser: 15-20 mm
Dicke: 2 mm +/- 0,025 mm
Planparallelität: +/- 0,05 mm
Oberflächenrauhigkeit : ca. 18 pm.
- WAK: Thermischer Ausdehnungskoeffizient der Glaskeramik gemäß ISO 6872 (2008), gemessen im Bereich von 100 bis 500°C)
- σ_{Biax}: Biaxiale Bruchfestigkeit, gemessen gemäß Dentalnorm ISO 6872 (2008)

Die Mengen der Kristallphasen wurden mittels der Rietveld-Methode bestimmt. Dazu wurden Pulver der jeweiligen Glaskeramik verwendet, die mit Al₂O₃ (Produkt-Name: Taimicron TM-DAR, Firma: Taimei Chemicals, Co. Ltd., Japan) als internem Standard in einem Verhältnis von 50 Gew.-% Glaskeramik zu 50 Gew.-% Al₂O₃ gemischt wurden. Diese Mischung wurde mit Aceton aufgeschlämmt, um eine möglichst gute Durchmischung zu erreichen. Anschliessend wurde die Mischung bei etwa 80°C getrocknet. Danach wurde mittels eines D8 Advance Diffraktometers der Firma Bruker ein Diffraktogramm im Bereich 10 bis 100° 20 mittels Cu_{Kα}-Strahlung und einer Schrittweite von 0,014° 20 aufgenommen. Dieses Diffraktogramm wurde dann mit der Software TOPAS der Firma Bruker ausgewertet, und die Phasenanteile wurden bestimmt. Für alle Diffraktogramme wurde eine Untergrenze für die Li₃PO₄-Kristallitgrösse von etwa 30nm verwendet.

Zur Erzeugung der Gläser und Glaskeramiken wurden zunächst die Ausgangsgläser im 100 bis 200 g Massstab aus üblichen Rohstoffen bei 1500°C oder 1400°C für eine Dauer von 1 bis 3 Stunden erschmolzen, wobei das Erschmelzen sehr gut ohne Bildung von Blasen oder Schlieren möglich war. Durch Eingießen der Ausgangsgläser in Wasser wurden Glasfritten hergestellt, die zur Homogenisierung anschließend ein zweites Mal bei 1500°C oder 1400°C für 1 Stunde geschmolzen wurden.

Eine erste Wärmebehandlung der Ausgangsgläser bei einer Temperatur von 460 bis 550°C führte zur Bildung von Gläsern mit Keimen. Diese keimhaltigen Gläser kristallisierten durch eine weitere Wärmebehandlung bei 760 bis 880°C zu Glaskeramiken mit Lithiumsilikat als Hauptkristallphase und Tiefquarz als weiterer Kristallphase, wie durch Röntgenbeugungsuntersuchungen festgestellt wurde. Es wurden daher Lithiumsilikat-Tiefquarz-Glaskeramiken erhalten.

### A) Massivglasblöcke

In den Beispielen 1-26, 28 und 31-34 wurden die Glaskeramiken aus Massivglasblöcken hergestellt. Dazu wurden die erhaltenen Glasgranulate erneut bei der Temperatur T_{S} für eine Dauer t_{S} aufgeschmolzen. Die erhaltenen Schmelzen des Ausgangsglases wurden anschließend in eine Graphitform gegossen, um Massivglasblöcke zu erzeugen. Diese Glasmonolithe wurden daraufhin bei der Temperatur T_{Kb} für eine Dauer t_{Kb} entspannt, wodurch Keimbildung stattfinden konnte. Die keimhaltigen Ausgangsgläser wurden dann für eine Dauer t_{C} auf eine Temperatur T_{C} erwärmt. Dadurch wurden Glaskeramiken mit Lithiumdisilikat als Hauptkristallphase und Tiefquarz als Nebenphase gebildet, wie durch Röntgenbeugungsuntersuchungen bei Raumtemperatur festgestellt werden konnte.

Es wird angenommen, dass bei dieser Verfahrensvariante eine Volumenkristallisation von Lithiumdisilikat und Tiefquarz stattgefunden hat.

### B) Pulverpresslinge

Im Beispiel 27 wurde die Glaskeramik aus Pulverpresslingen hergestellt. Dazu wurde das erhaltene Glassgranulat in einer Zirkonoxidmühle auf eine Korngrösse von < 90pm aufgemahlen. Ca. 4g dieses Pulvers wurde anschließend zu zylinderförmigen Rohlingen verpresst und in einem Sinterofen (Programat^{®} von Ivoclar Vivadent AG) bei der Temperatur T_{C} und einer Haltezeit von t_{C} zu dichten glaskeramischen Körpern gesintert. Durch die Sinterung wurde eine Glaskeramik mit Lithiummetasilikat als Hauptkristallphase sowie Lithiumdisilikat und Tiefquarz als Nebenphasen gebildet, wie durch Röntgenbeugungsuntersuchungen bei Raumtemperatur festgestellt werden konnte.

### C) Herstellung einer Dentalrestauration aus Blöcken gemäß A)

Die gemäß den Beispielen 1-26, 28 und 31-34 erzeugten Glaskeramikblöcke wurden in einer CAD/CAM-Einheit maschinell zu gewünschten Dentalrestaurationen, wie Kronen, verarbeitet. Dazu wurden die kristallisierten Blöcke mit einem geeigneten Halter versehen, und anschließend wurde ihnen in einer Schleifeinheit inLab MC XL der Firma Sirona Dental GmbH, Deutschland, die gewünschte Form gegeben. Für die Verarbeitung der erfindungsgemäßen Rohlinge konnten die gleichen Schleifparameter wie für kommerzielle e.max CAD-Blöcke, Ivoclar Vivadent, Liechtenstein, verwendet werden.

### D) Heißpressen der Glaskeramik

In Beispiel 19, für das T_{Press} und t_{Press} angegeben sind, wurde die Glaskeramik durch Heißpressen aus Massivglasblöcken hergestellt.

Dazu wurde das erhaltene Glasgranulat erneut bei der Temperatur T_{S} für eine Dauer t_{S} aufgeschmolzen. Die erhaltene Schmelze des Ausgangsglases wurde anschließend in eine vorgewärmte Stahlform gegossen, um Stäbe zu erzeugen. Diese monolithischen Glasstäbe wurden daraufhin bei einer Temperatur T_{Kb} für eine Dauer t_{Kb} entspannt, wodurch Keimbildung stattfinden konnte. Die Stäbe wurden dann zu kleinen Zylindern einer Masse von ungefähr 4 bis 6 g zersägt. Diese kleinen Zylinder wurden dann bei einer Temperatur T_{C} für eine Dauer von t_{C} kristallisiert. Die keimgebildeten und kristallisierten Zylinder wurden dann in einem Heißpressofen bei der Temperatur T_{Press} und einer Haltezeit von tₚᵣₑₛₛ zu einem Formkörper verpresst. Nach dem Heißpressen war eine

Glaskeramik mit Lithiumdisilikat als Hauptkristallphase und Tiefquarz als weitere Kristallphase entstanden, wie durch Röntgenbeugungsuntersuchungen des gebildeten Formkörpers bei Raumtemperatur festgestellt werden konnte.

### E) Sinterung eines keimgebildeten Glases

In Beispiel 29 wurde das Ausgangsglas bei 1500°C für 2h aufgeschmolzen und anschließend in Wasser abgeschreckt. Das erhaltene Glasgranulat wurde dann einer Temperatur T_{Kb} und einer Zeit t_{Kb} keimgebildet. Das keimgebildete Ausgangsglas wurde zu einem Pulver mit einer mittleren Korngröße von 20 µm zerkleinert. Aus diesem keimgebildeten Glaspulver wurde ein Prüfkörper zur Bestimmung der Wärmeausdehnung und zur Bestimmung der optischen Eigenschaften hergestellt und bei einer Temperatur von T_{C} und einer Zeit t_{C} kristallisiert und dichtgesintert. Nach dem Dichtsintern war eine Glaskeramik mit Lithiumdisilikat als Hauptkristallphase und Tiefquarz als weiterer Nebenphase entstanden, wie durch Röntgenbeugungsuntersuchungen des gebildeten Formkörpers bei Raumtemperatur festgestellt werden konnte.

**Tabelle I**

| **Beispiel Nr.** | **1** | **2** | **3** | **4** | **5** |
|---|---|---|---|---|---|
| Zusammensetzung | Gew.-% | Gew.-% | Gew.-% | Gew.-% | Gew.-% |
| SiO₂ | 74,3 | 73,3 | 72,0 | 72,0 | 74,9 |
| Li₂O | 11,2 | 12,6 | 13,3 | 12,3 | 10,7 |
| K₂O | 3,4 | 3,2 | 3,5 | 3,4 | 3,4 |
| Rb₂O | - | - | - | - | - |
| MgO | 4,4 | 1,4 | 4,5 | 4,4 | 4,4 |
| CaO | - | 1,9 | - | - | - |
| SrO | - | - | - | - | - |
| Al₂O₃ | 2,8 | 3,5 | 2,8 | 2,8 | 2,8 |
| Ga₂O₃ | - | - | - | - | - |
| Er₂O₃ | - | - | - | 0,1 | - |
| CeO₂ | - | - | - | 0,8 | - |
| V₂O₅ | - | - | - | 0,1 | - |
| P₂O₅ | 3,9 | 4,1 | 3,9 | 3,9 | 3,8 |
| F⁻ | - | - | - | - | - |
| Tb₄O₇ | - | - | - | 0,3 | - |
| T_{g} / °C | 471 | 465 | 469 | 463 | 471 |
| Tₛ / °C, tₛ / min | 1500, 120 | 1520, 120 | 1500, 120 | 1500, 120 | 1500, 120 |
| T_{Kb} / °C, t_{Kb} / min | 500, 30 | 480, 10 | 500, 30 | 500, 30 | 500, 30 |
| T_{c} / °C, t_{c} / min | 800, 30 | 800, 15 | 800, 30 | 810,20 | 800, 30 |
| Hauptkristallphase (Gew.-%) | Li₂Si₂O₅ (40,9) | Li₂Si₂O₅ | Li₂Si₂O₅ (51,3) | Li₂Si₂O₅ (43,4) | Li₂Si₂O₅ (36,2) |
| weitere Kristallphasen (Gew.-%) | Tiefquarz (17,5), Li₃PO₄ (6,3) | Tiefquarz, Li₃PO₄ | Tiefquarz (0,2), Li₃PO₄ (6,8) | Tiefquarz (4,4), Li₃PO₄ (6,0), | Tiefquarz (20,7), Li₃PO₄ (5,4) |
| σ_{Biax} /MPa | 464 | | | 376 | |
| CR-Wert | 71,83 | | | 71,45 | 71,21 |
| L* | 94,15 | | | 89,46 | 93,90 |
| a* | -0,45 | | | 0,48 | -0,40 |
| b* | 3,44 | | | 13,22 | 3,92 |
| WAK / 10⁻⁶K⁻¹ (100-500°C) | | | | | |

| **Beispiel Nr.** | **6** | **7** | **8** | **9** | **10** |
|---|---|---|---|---|---|
| Zusammensetzung | Gew.-% | Gew.-% | Gew.-% | Gew.-% | Gew.-% |
| SiO₂ | 72,3 | 72,6 | 70,1 | 73,0 | 75,6 |
| Li₂O | 12,0 | 11,7 | 11,3 | 11,4 | 10,2 |
| K₂O | 3,4 | 3,4 | - | 3,4 | 3,4 |
| Rb₂O | - | - | 6,5 | - | - |
| MgO | 4,4 | 4,4 | 4,2 | 4,4 | 4,3 |
| CaO | - | - | - | - | - |
| SrO | - | - | - | - | - |
| Al₂O₃ | 2,8 | 2,8 | 2,2 | 2,8 | 2,7 |
| Ga₂O₃ | - | - | - | - | - |
| Er₂O₃ | 0,1 | 0,1 | 0,1 | 0,1 | - |
| CeO₂ | 0,8 | 0,8 | 0,7 | 0,6 | - |
| V₂O₅ | 0,1 | 0,1 | 0,1 | 0,1 | - |
| P₂O₅ | 3,9 | 3,8 | 4,5 | 3,8 | 3,8 |
| F⁻ | - | - | - | - | - |
| Tb₄O₇ | 0,3 | 0,3 | 0,3 | 0,4 | - |
| T_{g}/°C | 469 | 473 | 472 | 470 | 480 |
| Tₛ / °C, tₛ / min | 1500, 120 | 1500, 120 | 1500, 120 | 1500, 120 | 1500, 120 |
| T_{Kb} / °C, t_{Kb}/ min | 480, 60 | 520, 10 | 480, 120 | 470, 10 | 500, 30 |
| T_{c} / °C, t_{c} / min | 800, 30 | 820, 10 | 800, 10 | 780, 30 | 800, 30 |
| Hauptkristallphase (Gew.-%) | Li₂Si₂O₅ (42,7) | Li₂Si₂O₅ (39,0) | Li₂Si₂O₅ (30,0) | Li₂Si₂O₅ (38,4) | Li₂Si₂O₅ (32,7) |
| weitere Kristallphasen (Gew.-%) | Tiefquarz (10,2), Li₃PO₄ (6,0) | Tiefquarz (12,1), Li₃PO₄ (6,0) | Tiefquarz (7,1), Li₃PO₄ (7,1) | Tiefquarz (14,8), Li₃PO₄ (5,6) | Tiefquarz (24,2), Li₃PO₄ (6,3) |
| σ_{Biax} /MPa | 371 | 395 | 456 | 326 | 347 |
| CR-Wert | 69,27 | 68,94 | 77,14 | 68,63 | 71,28 |
| L* | 89,78 | 89,68 | 90,06 | 90,29 | 94,07 |
| a* | 0,34 | 0,18 | -0,13 | 0,85 | -0,46 |
| b* | 13,65 | 13,9 | 9,07 | 11,17 | 3,46 |
| WAK / 10⁻⁶K⁻¹ (100-500°C) | 10,8 | 11,3 | | 11,5 | |

| **Beispiel Nr.** | **11** | **12** | **13** | **14** | **15** |
|---|---|---|---|---|---|
| Zusammensetzung | Gew.-% | Gew.-% | Gew.-% | Gew.-% | Gew.-% |
| SiO₂ | 72,9 | 72,2 | 70,2 | 72,4 | 70,4 |
| Li₂O | 11,3 | 11,6 | 12,5 | 10,9 | 12,1 |
| K₂O | 2,1 | 3,4 | 3,3 | 3,4 | 3,1 |
| Rb₂O | - | - | - | - | - |
| MgO | 1,8 | 4,4 | 1,6 | 4,3 | 3,4 |
| CaO | 1,8 | - | 2,3 | - | - |
| SrO | 3,3 | - | - | - | - |
| Al₂O₃ | 2,7 | 4,6 | 4,0 | 3,9 | 3,6 |
| Ga₂O₃ | - | - | - | - | 2,5 |
| Er₂O₃ | - | - | 0,2 | 0,2 | 0,1 |
| CeO₂ | - | - | 1,2 | 0,6 | 0,9 |
| V₂O₅ | - | - | 0,1 | 0,1 | 0,1 |
| P₂O₅ | 3,8 | 3,8 | 4,3 | 3,8 | 3,5 |
| F⁻ | 0,3 | - | - | - | - |
| Tb₄O₇ | - | - | 0,3 | 0,4 | 0,3 |
| T_{g}/°C | 453 | 477 | 464 | 472 | 462 |
| Tₛ / °C, tₛ / min | 1500, 120 | 1500, 120 | 1500, 120 | 1500, 120 | 1500, 120 |
| T_{Kb} / °C, t_{Kb}/ min | 460, 90 | 500, 30 | 500, 10 | 480, 40 | 540, 10 |
| T_{c} / °C, t_{c} / min | 800, 40 | 800, 30 | 800, 60 | 770, 60 | 790, 30 |
| Hauptkristallphase (Gew.-%) | Li₂Si₂O₅ (45,0) | Li₂Si₂O₅ (38,7) | Li₂Si₂O₅ (38,1) | Li₂Si₂O₅ (34,4) | Li₂Si₂O₅ (39,0) |
| weitere Kristallphasen (Gew.-%) | Tiefquarz (19,3), Li₃PO₄ (2,8), Ca₂Sr₃(PO₄)₃F (5,5) | Tiefquarz (13,4), Li₃PO₄ (5,4) | Tiefquarz (9,4), Li₃PO₄ (6,3) | Tiefquarz (17,4), Li₃PO₄ (5,2) | Tiefquarz (9,9), Li₃PO₄ (5,4) |
| σ_{Biax} /MPa | 397 | | 350 | 377 | 285 |
| CR-Wert | 70,06 | | 64,56 | 69,01 | 70,84 |
| L* | 89,22 | | 85,82 | 90,67 | 86,98 |
| a* | 0,50 | | 2,6 | 1,95 | 1,80 |
| b* | 5,87 | | 19,74 | 8,96 | 19,10 |
| WAK / 10⁻⁶K⁻¹ (100-500°C) | | | 10,6 | 10,8 | |

| **Beispiel Nr.** | **16** | **17** | **18** | **19** | **20** |
|---|---|---|---|---|---|
| Zusammensetzung | Gew.-% | Gew.-% | Gew.-% | Gew.-% | Gew.-% |
| SiO₂ | 69,2 | 71,5 | 71,0 | 74,7 | 70,0 |
| Li₂O | 11,5 | 10,5 | 10,0 | 9,8 | 10,5 |
| K₂O | 3,2 | 3,3 | 3,3 | 3,3 | 3,2 |
| Cs₂O | - | - | - | - | - |
| Rb₂O | - | - | - | - | - |
| MgO | 3,1 | 3,5 | 3,8 | 4,3 | 3,8 |
| CaO | - | - | - | - | - |
| SrO | - | - | - | - | - |
| ZnO | - | - | - | - | - |
| Al₂O₃ | 3,1 | 2,8 | 3,0 | 2,9 | 3,8 |
| Ga₂O₃ | - | - | - | - | - |
| La₂O₃ | - | - | 3,4 | - | - |
| Y₂O₃ | - | 2,9 | - | - | - |
| In₂O₃ | 4,7 | - | - | - | - |
| Er₂O₃ | 0,2 | 0,1 | 0,1 | 0,1 | 0,2 |
| ZrO₂ | - | - | - | - | - |
| SnO₂ | - | - | - | - | - |
| CeO₂ | 1,0 | 0,6 | 1,2 | 0,8 | 0,5 |
| MnO₂ | - | - | - | - | - |
| V₂O₅ | 0,1 | 0,1 | 0,1 | 0,1 | 0,1 |
| Ta₂O₅ | - | - | - | - | 3,8 |
| P₂O₅ | 3,5 | 4,3 | 3,7 | 3,6 | 3,7 |
| F⁻ | - | - | - | - | - |
| Tb₄O₇ | 0,4 | 0,4 | 0,4 | 0,4 | 0,4 |
| T_{g}/°C | 483 | 477 | 478 | 467 | 482 |
| Tₛ / °C, tₛ / min | 1500, 120 | 1500, 120 | 1500, 120 | 1500, 120 | 1500, 120 |
| T_{Kb} / °C, t_{Kb}/ min | 550, 30 | 480, 10 | 500, 40 | 470, 60 | 500, 20 |
| T_{c} / °C, t_{c} / min | 770, 20 | 760, 10 | 760, 20 | 750, 30 | 760, 30 |
| Tₚᵣₑₛₛ / °C, tₚᵣₑₛₛ / °C | | | | 870, 25 | |
| Hauptkristallphase (Gew.-%) | Li₂Si₂O₅ (32,4) | Li₂Si₂O₅ (27,0) | Li₂Si₂O₅ (27,3) | Li₂Si₂O₅ (28,8) | Li₂Si₂O₅ (29,1) |
| weitere Kristallphasen (Gew.-%) | Tiefquarz (9,8), Li₃PO₄ (4,9), | Tiefquarz (19,9), Li₃PO₄ (6,0) | Tiefquarz (18,6), Li₃PO₄ (5,1) | Tiefquarz (24,3), Li₃PO₄ (3,8) | Tiefquarz (14,8), Li₃PO₄ (4,4) |
| σ_{Biax} /MPa | 299 | 290 | 320 | | 367 |
| CR-Wert | 57,60 | 56,69 | 46,84 | 64,29 | 63,10 |
| L* | 85,77 | 90,49 | 84,38 | 90,6 | 89,91 |
| a* | 0,16 | -0,50 | 0,05 | 0,38 | 1,70 |
| b* | 19,18 | 9,68 | 26,72 | 12,76 | 9,43 |
| WAK / 10⁻⁶K⁻¹ (100-500°C) | | | | 12,8 | |

| **Beispiel Nr.** | **21** | **22** | **23** | **24** | **25** |
|---|---|---|---|---|---|
| Zusammensetzung | Gew.-% | Gew.-% | Gew.-% | Gew.-% | Gew.-% |
| SiO₂ | 72,9 | 68,8 | 69,5 | 73,2 | 73,7 |
| Li₂O | 12,5 | 11,4 | 11,5 | 11,7 | 11,5 |
| K₂O | 3,5 | 3,3 | 3,3 | 0,8 | 3,3 |
| Cs₂O | - | - | - | 1,3 | - |
| Rb₂O | - | - | - | 1,3 | - |
| MgO | 4,4 | 3,2 | 1,1 | 2,9 | - |
| CaO | - | - | 1,5 | - | - |
| SrO | - | - | 2,4 | - | 3,6 |
| ZnO | - | - | 1,8 | - | - |
| Al₂O₃ | 2,8 | 3,2 | 2,7 | 2,7 | 2,5 |
| Ga₂O₃ | - | - | - | - | - |
| La₂O₃ | - | - | - | - | - |
| Y₂O₃ | - | - | - | - | - |
| In₂O₃ | - | - | - | - | - |
| Er₂O₃ | - | 0,2 | 0,2 | 0,1 | 0,1 |
| ZrO₂ | - | 2,1 | - | - | - |
| SnO₂ | - | 2,6 | - | - | - |
| CeO₂ | - | 1,1 | 1,8 | 1,5 | 1,5 |
| MnO₂ | - | - | - | 0,1 | - |
| V₂O₅ | - | 0,1 | 0,2 | 0,2 | 0,1 |
| Ta₂O₅ | - | - | - | - | - |
| P₂O₅ | 3,9 | 3,6 | 3,7 | 3,8 | 3,3 |
| F⁻ | - | - | - | - | - |
| Tb₄O₇ | - | 0,4 | 0,3 | 0,4 | 0,4 |
| T_{g}/°C | 473 | 483 | 461 | 467 | 472 |
| Tₛ / °C, tₛ / min | 1500, 120 | 1500, 120 | 1500, 120 | 1500, 120 | 1500, 120 |
| T_{Kb} / °C, t_{Kb}/ min | 500, 30 | 490,30 | 480, 30 | 500, 20 | 500, 70 |
| T_{c} / °C, t_{c} / min | 800, 30 | 770, 40 | 800, 10 | 820, 30 | 830,40 |
| Tₚᵣₑₛₛ / °C, tₚᵣₑₛₛ / °C | | | | | |
| Hauptkristallphase (Gew.-%) | Li₂Si₂O₅ (48,0) | Li₂Si₂O₅ | Li₂Si₂O₅ | Li₂Si₂O₅ | Li₂Si₂O₅ (37,3) |
| weitere Kristallphasen (Gew.-%) | Tiefquarz (6,5), Li₃PO₄ (6,6), | Tiefquarz, Li₃PO₄ | Tiefquarz, Li₃PO₄ | Tiefquarz, Li₃PO₄ | Tiefquarz (14,7), Li₃PO₄ (2,1) |
| σ_{Biax}/MPa | | | 487 | | |
| CR-Wert | 74,98 | | 53,15 | | |
| L* | 94,16 | | 79,74 | | |
| a* | -0,62 | | 3,58 | | |
| b* | 3,40 | | 34,15 | | |
| WAK / 10⁻⁶K⁻¹ (100-500°C) | | | | | |

| **Beispiel Nr.** | **26** | **27** | **28** | **29** | |
|---|---|---|---|---|---|
| Zusammensetzung | Gew.-% | Gew.-% | Gew.-% | Gew.-% | |
| SiO₂ | 73,0 | 74,8 | 68,9 | 75,8 | |
| Li₂O | 11,7 | 13,3 | 12,2 | 10,5 | |
| K₂O | 3,4 | 3,6 | 3,3 | 3,4 | |
| Cs₂O | - | - | - | - | |
| Rb₂O | - | - | - | - | |
| MgO | 4,4 | 1,7 | 1,4 | 3,6 | |
| CaO | - | 2,4 | 2,1 | - | |
| SrO | - | - | - | - | |
| ZnO | - | - | - | - | |
| Al₂O₃ | 3,7 | 4,2 | 3,9 | 2,9 | |
| Ga₂O₃ | - | - | - | - | |
| La₂O₃ | - | - | - | - | |
| Y₂O₃ | - | - | - | - | |
| In₂O₃ | - | - | - | - | |
| Er₂O₃ | - | - | - | - | |
| ZrO₂ | - | - | - | - | |
| SnO₂ | - | - | - | - | |
| CeO₂ | - | - | - | - | |
| MnO₂ | - | - | - | - | |
| V₂O₅ | - | - | - | - | |
| Ta₂O₅ | - | - | - | - | |
| P₂O₅ | 3,8 | - | 8,2 | 3,8 | |
| F⁻ | - | - | - | - | |
| Tb₄O₇ | - | - | - | - | |
| T_{g}/°C | | 457 | 471 | 469 | |
| Tₛ / °C, tₛ / min | 1500, 120 | 1500, 180 | 1500, 150 | 1500, 120 | |
| T_{Kb} / °C, t_{Kb} / min | 500, 30 | - | 490, 10 | 500, 30 | |
| T_{c} / °C, t_{c} / min | 800, 30 | 780, 10 | 800, 30 | 880, 1 | |
| Tₚᵣₑₛₛ / °C, tₚᵣₑₛₛ / °C | | | | | |
| Hauptkristallphase (Gew.-%) | Li₂Si₂O₅ (45,0) | Li₂SiO₃, | Li₂Si₂O₅, | Li₂Si₂O₅, | |
| weitere Kristallphasen (Gew.-%) | Tiefquarz (12,8), Li₃PO₄ (6,0), | Tiefquarz, Li₂Si₂O₅, Li₃PO₄ | Tiefquarz, Li₃PO₄ | Tiefquarz, Li₃PO₄ | |
| σ_{Biax} /MPa | | | 320 | | |
| CR-Wert | | | 67,66 | 76,6 | |
| L* | | | 91,17 | 94 | |
| a* | | | 0,08 | -0,21 | |
| b* | | | 4,69 | 2,55 | |
| WAK / 10⁻⁶K⁻¹ (100-500°C) | | | | 12,3 | |

| **Beispiel Nr.** | **30** | **31** | **32** | **33** | **34** |
|---|---|---|---|---|---|
| Zusammensetzung | Gew.-% | Gew.-% | Gew.-% | Gew.-% | Gew.% |
| SiO₂ | 64,4 | 67,1 | 69,8 | 71,6 | 59,7 |
| Li₂O | 14,6 | 13,3 | 11,9 | 11,1 | 12,9 |
| Na₂O | - | - | 1,6 | 1,9 | - |
| K₂O | 3,3 | 3,3 | - | - | 3,2 |
| Cs₂O | - | - | 5,0 | 3,7 | - |
| Rb₂O | - | - | - | - | - |
| MgO | - | - | 3,9 | 3,2 | 0,2 |
| CaO | - | - | - | - | - |
| SrO | - | - | - | - | - |
| ZnO | 8,7 | 8,6 | - | - | 8,3 |
| Al₂O₃ | 2,7 | 2,7 | 2,7 | 2,7 | 2,6 |
| Ga₂O₃ | - | - | - | - | - |
| La₂O₃ | - | - | - | - | - |
| Er₂O₃ | - | - | 0,1 | 0,1 | - |
| ZrO₂ | - | - | - | - | - |
| SnO₂ | - | - | - | - | - |
| CeO₂ | - | - | 0,8 | 1,5 | - |
| GeO₂ | - | - | - | - | 8,9 |
| MnO₂ | - | - | - | - | - |
| V₂O₅ | - | - | 0,1 | 0,1 | - |
| Ta₂O₅ | - | - | - | - | - |
| P₂O₅ | 6,3 | 5,0 | 3,8 | 3,7 | 4,2 |
| F⁻ | - | - | - | - | - |
| Tb₄O₇ | - | - | 0,3 | 0,4 | - |
| T_{g} / °C | 455 | 459 | 458 | 463 | |
| Tₛ / °C, tₛ / min | 1500, 120 | 1500, 120 | 1500, 120 | 1500, 120 | 1400, 120 |
| T_{Kb} / °C, t_{Kb}/ min | 500, 30 | 500, 30 | 500, 30 | 500, 30 | 500, 30 |
| T_{c} / °C, t_{c} / min | 840, 30 | 850, 30 | 800, 30 | 800, 30 | 820, 30 |
| Tₚᵣₑₛₛ / °C, tₚᵣₑₛₛ / °C | | | | | |
| Hauptkristallphase (Gew.-%) | Li₂Si₂O₅, | Li₂Si₂O₅, | Li₂Si₂O₅, | Li₂Si₂O₅, (33,8) | Li₂Si₂O₅, |
| weitere Kristallphasen (Gew.-%) | Tiefquarz, Li₃PO₄ | Tiefquarz, Li₃PO₄ | Tiefquarz, Li₃PO₄, Cs_{0.809}AlSi₅O₁₂ | Tiefquarz, (15,6) Li₃PO₄, (5,8) Cs_{0.809}AlSi₅O₁₂ (10,0) | Tiefquarz, Li₃PO₄ |
| σ_{Biax} /MPa | 458 | 485 | 516 | 368 | - |
| CR-Wert | 90,90 | 86,57 | 73,4 | 73,54 | - |
| L* | 95,87 | 95,52 | 90,36 | 82,05 | - |
| a* | -0,24 | -0,24 | 0,32 | 4,63 | - |
| b* | 0,84 | 0,66 | 11,49 | 26,02 | - |
| WAK / 10⁻⁶K⁻¹ (100-500°C) | | | | | |

## Patentansprüche

1. Verwendung von Lithiumsilikat-Tiefquarz-Glaskeramik, die 1,0 bis 8,0 Gew.-% Oxid einwertiger Elemente Me^{I}₂O ausgewählt aus der Gruppe von K₂O, Na₂O, Rb₂O, Cs₂O und Mischungen davon und 1,0 bis 6,0 Gew.-% MgO enthält und die Lithiumsilikat als Hauptkristallphase und Tiefquarz als weitere Kristallphase enthält, als Dentalmaterial,
ausgenommen Glaskeramik mit der folgenden Zusammensetzung
| Komponente | Gew.-% |
|---|---|
| SiO₂ | 65,7 |
| Li₂O | 13,3 |
| CaO | 4,0 |
| MgO | 2,9 |
| K₂O | 3,2 |
| Al₂O₃ | 3,2 |
| P₂O₃ | 7, 7, |
die Lithiumdisilikat als Hauptkristallphase und CaMgSi₂O₆, Tiefquarz und Li₃PO₄ als weitere Kristallphasen enthält und dadurch hergestellt ist, dass ein Ausgangsglas in einem Platintiegel bei 1500°C für eine Dauer von 120 min erschmolzen wird, durch Eingießen des erschmolzenen Ausgangsglases in Wasser eine Glasfritte hergestellt wird, die Glasfritte mit einer Schwingmühle und einer Zirkonoxidschwingmühle auf eine mittlere Korngröße von <90 pm, bezogen auf die Anzahl der Teilchen, gemahlen wird und das gemahlene Glaspulver uniaxial zu einem kleinen Zylinder verpresst und in einem Ofen bei 840°C für 5 min kristallisiert und gesintert wird.

2. Verwendung von Lithiumsilikat-Tiefquarz-Glaskeramik, die 1,0 bis 8,0 Gew.-% Oxid einwertiger Elemente Me^{I}₂O ausgewählt aus der Gruppe von K₂O, Na₂O, Rb₂O, Cs₂O und Mischungen davon und 1,0 bis 6,0 Gew.-% MgO enthält und die Lithiumsilikat als Hauptkristallphase und Tiefquarz als weitere Kristallphase enthält, als Dentalmaterial,
wobei die Glaskeramik
68,0 bis 79,0 Gew.-% SiO₂ oder
0 bis 6,0 Gew.-% P₂O₅ oder
3,1 bis 6,0 Gew.-% MgO oder
0 bis 3,0 Gew.-% CaO
enthält.

3. Verwendung nach Anspruch 1 oder 2, bei der die Glaskeramik 59,0 bis 79,0, bevorzugt 64,0 bis 78,0 und besonders bevorzugt 64,0 bis 76,0 Gew.-% SiO₂ oder 68,0 bis 79,0, bevorzugt 69,0 bis 78,0 und besonders bevorzugt 70,0 bis 76,0 Gew.-% SiO₂ enthält.

4. Verwendung nach einem der Ansprüche 1 bis 3, bei der die Glaskeramik 8,0 bis 15,0, bevorzugt 9,0 bis 14,0 und besonders bevorzugt 10,0 bis 13,5 Gew.-% Li₂O enthält.

5. Verwendung nach einem der Ansprüche 1 bis 4, bei der die Glaskeramik 0 bis 9,0, bevorzugt 2,0 bis 6,0 und besonders bevorzugt 3,0 bis 5,0 Gew.-% P₂O₅ enthält.

6. Verwendung nach einem der Ansprüche 1 bis 5, bei der die Glaskeramik 2,0 bis 7,0 Gew.-% Oxid einwertiger Elemente Me^{I}₂O ausgewählt aus der Gruppe von K₂O, Na₂O, Rb₂O, Cs₂O und Mischungen davon enthält.

7. Verwendung nach einem der Ansprüche 1 bis 6, bei der die Glaskeramik 0 bis 5,0, bevorzugt 1,0 bis 4,0 und besonders bevorzugt 2,0 bis 3,5 Gew.-% K₂O enthält.

8. Verwendung nach einem der Ansprüche 1 bis 7, bei der die Glaskeramik 1,0 bis 9,0, bevorzugt 2,0 bis 8,0 und besonders bevorzugt 3,0 bis 7,0 Gew.-% Oxid zweiwertiger Elemente Me^{II}O ausgewählt aus der Gruppe von CaO, MgO, SrO, ZnO und Mischungen davon enthält.

9. Verwendung nach einem der Ansprüche 1 bis 8, bei der die Glaskeramik 1,5 bis 6,0, bevorzugt 2,0 bis 5,5, besonders bevorzugt 3,1 bis 5,5 und ganz besonders bevorzugt 3,4 bis 5,0 Gew.-% MgO enthält.

10. Verwendung nach einem der Ansprüche 1 bis 9, bei der die Glaskeramik 0 bis 8,0, bevorzugt 1,0 bis 7,0 und besonders bevorzugt 2,0 bis 6,5 Gew.-% Oxid dreiwertiger Elemente Me^{III}₂O₃ ausgewählt aus der Gruppe von Al₂O₃, B₂O₃, Y₂O₃, La₂O₃, Ga₂O₃, In₂O₃ und Mischungen davon enthält.

11. Verwendung nach einem der Ansprüche 1 bis 10, bei der die Glaskeramik 1,0 bis 6,0 und bevorzugt 2,0 bis 5,0 Gew.-% Al₂O₃ enthält.

12. Verwendung nach einem der Ansprüche 1 bis 11, bei der die Glaskeramik SiO₂ und Li₂O in einem Molverhältnis im Bereich von 2,2 bis 4,1, bevorzugt 2,2 bis 3,8 und besonders bevorzugt 2,2 bis 3,5 enthält.

13. Verwendung nach einem der Ansprüche 1 bis 12, bei der die Glaskeramik Lithiumdisilikat oder Lithiummetasilikat als Hauptkristallphase und bevorzugt Lithiumdisilikat als Hauptkristallphase enthält.

14. Verwendung nach einem der Ansprüche 1 bis 13, bei der die Glaskeramik mindestens 20 Gew.-%, bevorzugt 25 bis 55 Gew.-% und besonders bevorzugt 30 bis 55 Gew.-% Lithiumdisilikat-Kristalle aufweist.

15. Verwendung nach einem der Ansprüche 1 bis 14, bei der die Glaskeramik 0,2 bis 28 Gew.-% und bevorzugt 0,2 bis 25 Gew.-% Tiefquarz-Kristalle aufweist.

16. Verwendung von Ausgangsglas, das die Komponenten der Glaskeramik nach einem der Ansprüche 1 bis 12 und Keime für die Kristallisation von Lithiummetasilikat, Lithiumdisilikat und/oder Tiefquarz enthält, als Dentalmaterial.

17. Verwendung nach einem der Ansprüche 1 bis 16, wobei die Glaskeramik und das Ausgangsglas in Form von einem Pulver, einem Granulat, einem Rohling oder einer dentalen Restauration vorliegen.

18. Verwendung nach einem der Ansprüche 1 bis 17 zur Beschichtung dentaler Restaurationen und besonders bevorzugt zur Herstellung dentaler Restaurationen.

19. Verwendung zur Herstellung dentaler Restaurationen nach Anspruch 18, wobei der Glaskeramik durch Verpressen oder maschinelle Bearbeitung die Form der gewünschten dentalen Restauration, insbesondere Brücke, Inlay, Onlay, Veneer, Abutment, Teilkrone, Krone oder Schale, gegeben wird.

20. Verfahren zur Herstellung einer dentalen Restauration, insbesondere Brücke, Inlay, Onlay, Veneer, Abutment, Teilkrone, Krone oder Schale, bei dem der Glaskeramik gemäß einem der Ansprüche 1 bis 15 durch Verpressen oder maschinelle Bearbeitung, insbesondere im Rahmen eines CAD/CAM-Verfahrens, die Form der gewünschten dentalen Restauration gegeben wird.

21. Verfahren zur Herstellung von Lithiumsilikat-Tiefquarz-Glaskeramik, die 1,0 bis 8,0 Gew.-% Oxid einwertiger Elemente Me^{I}₂O ausgewählt aus der Gruppe von K₂O, Na₂O, Rb₂O, Cs₂O und Mischungen davon und 1,0 bis 6,0 Gew.-% MgO enthält und die Lithiumsilikat als Hauptkristallphase und Tiefquarz als weitere Kristallphase enthält, bei dem
(a) ein Ausgangsglas, das die Komponenten der Glaskeramik enthält, einer Wärmebehandlung bei einer Temperatur von 400 bis 600°C für 5 bis 120 min unterworfen wird, um Ausgangsglas mit Keimen zu bilden, und
(b) das Ausgangsglas mit Keimen einer Wärmebehandlung bei einer Temperatur von 700 bis 900°C für 5 bis 120 min unterworfen wird, um die Lithiumsilikat-Tiefquarz-Glaskeramik zu bilden.

## Claims

1. Use of lithium silicate-low quartz glass ceramic, which comprises 1.0 to 8.0 wt.-% oxide of monovalent elements Me^{I}₂O selected from the group of K₂O, Na₂O, Rb₂O, Cs₂O and mixtures thereof and 1.0 to 6.0 wt.-% of MgO and which comprises lithium silicate as main crystal phase and low quartz as further crystal phase, as dental material,
with the exception of glass ceramic having the following composition
| Component | wt.-% |
|---|---|
| SiO₂ | 65.7 |
| Li₂O | 13.3 |
| CaO | 4.0 |
| MgO | 2.9 |
| K₂O | 3.2 |
| Al₂O₃ | 3.2 |
| P₂O₃ | 7.7, |
which comprises lithium disilicate as main crystal phase and CaMgSi₂O₆, low quartz and Li₃PO₄ as further crystal phases and is prepared by melting a starting glass in a platinum crucible at 1500°C for a period of 120 min, producing a glass frit by pouring the melted starting glass into water, grinding the glass frit with a vibrating mill and a zirconia vibrating mill to an average particle size of <90 pm, based on the number of particles, and uniaxially pressing the ground glass powder into a small cylinder and crystallizing and sintering in a furnace at 840°C for 5 min.

2. Use of lithium silicate-low quartz glass ceramic, which comprises 1.0 to 8.0 wt.-% oxide of monovalent elements Me¹₂0 selected from the group of K₂O, Na₂O, Rb₂O, Cs₂O and mixtures thereof and 1.0 to 6.0 wt.-% of MgO and which comprises lithium silicate as main crystal phase and low quartz as further crystal phase, as dental material,
wherein the glass ceramic comprises
68.0 to 79.0 wt.-% SiO₂ or
0 to 6.0 wt.-% P₂O₅ or
3.1 to 6.0 wt.-% MgO or
0 to 3.0 wt.-% CaO.

3. Use according to claim 1 or 2, wherein the glass ceramic comprises 59.0 to 79.0, preferably 64.0 to 78.0 and particularly preferably 64.0 to 76.0 wt.-% SiO₂ or comprises 68.0 to 79.0, preferably 69.0 to 78.0 and particularly preferably 70.0 to 76.0 wt.-% SiO₂.

4. Use according to any one of claims 1 to 3, wherein the glass ceramic comprises 8.0 to 15.0, preferably 9.0 to 14.0 and particularly preferably 10.0 to 13.5 wt.-% Li₂O.

5. Use according to any one of claims 1 to 4, wherein the glass ceramic comprises 0 to 9.0, preferably 2.0 to 6.0 and particularly preferably 3.0 to 5.0 wt.-% P₂O₅.

6. Use according to any one of claims 1 to 5, wherein the glass ceramic comprises 2.0 to 7.0 wt.-% oxide of monovalent elements Me^{I}₂O selected from the group of K₂O, Na₂O, Rb₂O, Cs₂O and mixtures thereof.

7. Use according to any one of claims 1 to 6, wherein the glass ceramic comprises 0 to 5.0, preferably 1.0 to 4.0 and particularly preferably 2.0 to 3.5 wt.-% K₂O.

8. Use according to any one of claims 1 to 7, wherein the glass ceramic comprises 1.0 to 9.0, preferably 2.0 to 8.0 and particularly preferably 3.0 to 7.0 wt.-% oxide of divalent elements Me^{II}O selected from the group of CaO, MgO, SrO, ZnO and mixtures thereof.

9. Use according to any one of claims 1 to 8, wherein the glass ceramic comprises 1.5 to 6.0, preferably 2.0 to 5.5, particularly preferably 3.1 to 5.5 and quite particularly preferably 3.4 to 5.0 wt.-% MgO.

10. Use according to any one of claims 1 to 9, wherein the glass ceramic comprises 0 to 8.0, preferably 1.0 to 7.0 and particularly preferably 2.0 to 6.5 wt.-% oxide of trivalent elements Me^{III}₂O₃ selected from the group of Al₂O₃, B₂O₃, Y₂O₃, La₂O₃, Ga₂O₃, In₂O₃ and mixtures thereof.

11. Use according to any one of claims 1 to 10, wherein the glass ceramic comprises 1.0 to 6.0 and preferably 2.0 to 5.0 wt.-% Al₂O₃.

12. Use according to any one of claims 1 to 11, wherein the glass ceramic comprises SiO₂ and Li₂O in a molar ratio in the range of 2.2 to 4.1, preferably 2.2 to 3.8 and particularly preferably 2.2 to 3.5.

13. Use according to any one of claims 1 to 12, wherein the glass ceramic comprises lithium disilicate or lithium metasilicate as main crystal phase and preferably comprises lithium disilicate as main crystal phase.

14. Use according to any one of claims 1 to 13, wherein the glass ceramic comprises at least 20 wt.-%, preferably 25 to 55 wt.-% and particularly preferably 30 to 55 wt.-% lithium disilicate crystals.

15. Use according to any one of claims 1 to 14, wherein the glass ceramic comprises 0.2 to 28 wt.-% and preferably 0.2 to 25 wt.-% low quartz crystals.

16. Use of starting glass, which comprises the components of the glass ceramic according to any one of claims 1 to 12 and nuclei for the crystallization of lithium metasilicate, lithium disilicate and/or low quartz, as dental material.

17. Use according to any one of claims 1 to 16, wherein the glass ceramic and the starting glass are in the form of a powder, a granulate, a blank or a dental restoration.

18. Use according to any one of claims 1 to 17 for coating dental restorations and particularly preferably for the preparation of dental restorations.

19. Use for the preparation of dental restorations according to claim 18, wherein the glass ceramic is given the shape of the desired dental restoration, in particular bridge, inlay, onlay, veneer, abutment, partial crown, crown or facet, by pressing or machining.

20. Process for the preparation of a dental restoration, in particular bridge, inlay, onlay, veneer, abutment, partial crown, crown or facet, wherein the glass ceramic according to any one of claims 1 to 15 is given the shape of the desired dental restoration by pressing or machining, in particular as part of a CAD/CAM method.

21. Process for the preparation of lithium silicate-low quartz glass ceramic, which comprises 1.0 to 8.0 wt.-% oxide of monovalent elements Me^{I}₂O selected from the group of K₂O, Na₂O, Rb₂O, Cs₂O and mixtures thereof and 1.0 to 6.0 wt.-% MgO and which comprises lithium silicate as main crystal phase and low quartz as further crystal phase, wherein
(a) a starting glass comprising the components of the glass ceramic is subjected to a heat treatment at a temperature of 400 to 600°C for 5 to 120 min in order to form starting glass with nuclei, and
(b) the starting glass with nuclei is subjected to a heat treatment at a temperature of 700 to 900°C for 5 to 120 min in order to form the lithium silicate-low quartz glass ceramic.

## Revendications

1. Utilisation d'une vitrocéramique de silicate de lithium et de quartz alpha, qui contient de 1,0 à 8,0 % en poids d'oxyde(s) d'élément(s) monovalent(s) Me^{I}₂O choisi(s) dans l'ensemble constitué par K₂O, Na₂O, Rb₂O et Cs₂O et leurs mélanges et de 1,0 à 6,0 % en poids de MgO, et qui comprend du silicate de lithium en tant que principale phase cristalline et du quartz alpha en tant qu'autre phase cristalline, en tant que matériau dentaire,
à l'exception d'une vitrocéramique qui présente la composition suivante :
| Composant | % en poids |
|---|---|
| SiO₂ | 65,7 |
| Li₂O | 13,3 |
| CaO | 4,0 |
| MgO | 2,9 |
| K₂O | 3,2 |
| Al₂O₃ | 3,2 |
| P₂O₅ | 7,7 , |
qui comprend du silicate de lithium en tant que principale phase cristalline et du CaMgSi₂O₆, du quartz alpha et du Li₃PO₄ en tant qu'autres phases cristallines et qu'on a préparée en faisant fondre un verre de départ dans un creuset en platine, à 1500 °C durant 120 minutes, en fabriquant une plaque de verre fritté en versant le verre de départ fondu dans de l'eau, en broyant cette plaque de verre fritté, avec un broyeur vibrant et un vibro-broyeur en zircone, jusqu'à obtenir une taille moyenne de grain, rapportée au nombre des particules, de moins de 90 µm et en comprimant la poudre de verre broyé, en mode uniaxe, en un petit cylindre qu'on a soumis à une cristallisation et à un frittage dans un four, à 840 °C durant 5 minutes.

2. Utilisation d'une vitrocéramique de silicate de lithium et de quartz alpha, qui contient de 1,0 à 8,0 % en poids d'oxyde(s) d'élément(s) monovalent(s) Me^{I}₂O choisi(s) dans l'ensemble constitué par K₂O, Na₂O, Rb₂O et Cs₂O et leurs mélanges et de 1,0 à 6,0 % en poids de MgO, et qui comprend du silicate de lithium en tant que principale phase cristalline et du quartz alpha en tant qu'autre phase cristalline, en tant que matériau de dentisterie,
laquelle vitrocéramique contient
- de 68,0 à 79,0 % en poids de SiO₂
- ou de 0 à 6,0 % en poids de P₂O₅,
- ou de 3,1 à 6,0 % en poids de MgO,
- ou de 0 à 3,0 % en poids de CaO.

3. Utilisation conforme à la revendication 1 ou 2, dans laquelle la vitrocéramique contient de 59,0 à 79,0, de préférence de 64,0 à 78,0 et en particulier de 64,0 à 76,0 % en poids de SiO₂ ou bien de 68,0 à 79,0, de préférence de 69,0 à 78,0 et en particulier de 70,0 à 76,0 % en poids de SiO₂.

4. Utilisation conforme à l'une des revendications 1 à 3, dans laquelle la vitrocéramique contient de 8,0 à 15,0, de préférence de 9,0 à 14,0 et en particulier de 10,0 à 13,5 % en poids de Li₂O.

5. Utilisation conforme à l'une des revendications 1 à 4, dans laquelle la vitrocéramique contient de 0 à 9,0, de préférence de 2,0 à 6,0 et en particulier de 3,0 à 5,0 % en poids de P₂O₅.

6. Utilisation conforme à l'une des revendications 1 à 5, dans laquelle la vitrocéramique contient de 2,0 à 7,0 % en poids d'oxyde(s) d'élément(s) monovalent(s) Me^{I}₂O choisi(s) dans l'ensemble constitué par K₂O, Na₂O, Rb₂O et Cs₂O et leurs mélanges.

7. Utilisation conforme à l'une des revendications 1 à 6, dans laquelle la vitrocéramique contient de 0 à 5,0, de préférence de 1,0 à 4,0 et en particulier de 2,0 à 3,5 % en poids de K₂O.

8. Utilisation conforme à l'une des revendications 1 à 7, dans laquelle la vitrocéramique contient de 1,0 à 9,0, de préférence de 2,0 à 8,0 et en particulier de 3,0 à 7,0 % en poids d'oxyde(s) d'élément(s) divalent(s) Me^{II}O choisi(s) dans l'ensemble constitué par CaO, MgO, SrO et ZnO et leurs mélanges.

9. Utilisation conforme à l'une des revendications 1 à 8, dans laquelle la vitrocéramique contient de 1,5 à 6,0, de préférence de 2,0 à 5,5, en particulier de 3,1 à 5,5 et tout particulièrement de 3,4 à 5,0 % en poids de MgO.

10. Utilisation conforme à l'une des revendications 1 à 9, dans laquelle la vitrocéramique contient de 0 à 8,0, de préférence de 1,0 à 7,0 et en particulier de 2,0 à 6,5 % en poids d'oxyde(s) d'élément(s) trivalent(s) Me^{III}₂O₃ choisi(s) dans l'ensemble constitué par Al₂O₃, B₂O₃, Y₂O₃, La₂O₃ Ga₂O₃ et In₂O₃, et leurs mélanges.

11. Utilisation conforme à l'une des revendications 1 à 10, dans laquelle la vitrocéramique contient de 1,0 à 6,0 et de préférence de 2,0 à 5,0 % en poids de Al₂O₃.

12. Utilisation conforme à l'une des revendications 1 à 11, dans laquelle la vitrocéramique contient SiO₂ et Li₂O en un rapport molaire situé dans l'intervalle allant de 2,2 à 4,1, de préférence de 2,2 à 3,8 et en particulier de 2,2 à 3,5.

13. Utilisation conforme à l'une des revendications 1 à 12, dans laquelle la vitrocéramique comprend du disilicate de lithium ou du métasilicate de lithium en tant que principale phase cristalline, et de préférence, comprend du disilicate de lithium en tant que principale phase cristalline,

14. Utilisation conforme à l'une des revendications 1 à 13, dans laquelle la vitrocéramique comprend au moins 20 % en poids, de préférence de 25 à 55 % en poids et en particulier de 30 à 55 % en poids de cristaux de disilicate de lithium.

15. Utilisation conforme à l'une des revendications 1 à 14, dans laquelle la vitrocéramique comprend de 0,2 à 28 % en poids et de préférence de 0,2 à 25 % en poids de cristaux de quartz alpha.

16. Utilisation d'un verre de départ qui contient des composants de vitrocéramique conformes à l'une des revendications 1 à 12 et des germes pour la cristallisation de métasilicate de lithium, de disilicate de lithium et/ou du quartz alpha, en tant que matériau dentaire.

17. Utilisation conforme à l'une des revendications 1 à 16, dans laquelle la vitrocéramique et le verre de départ se présente sous la forme d'une poudre, d'un granulat, d'une pièce brute ou d'une pièce de restauration dentaire.

18. Utilisation conforme à l'une des revendications 1 à 17, pour le revêtement de pièces de restauration dentaire et en particulier pour la fabrication de pièces de restauration dentaire.

19. Utilisation pour la fabrication de pièces de restauration dentaire, conforme à la revendication 18, dans laquelle on donne à la vitrocéramique, par compression ou par usinage à la machine, la forme de la pièce de restauration dentaire voulue, en particulier bridge, inlay, onlay, facette, pilier, couronne partielle, couronne ou coquille,

20. Procédé de fabrication d'une pièce de restauration dentaire, en particulier bridge, inlay, onlay, facette, pilier, couronne partielle, couronne ou coquille, dans lequel on donne à une vitrocéramique du type indiqué dans l'une des revendications 1 à 15, par compression ou par usinage à la machine, en particulier dans le cadre d'un procédé CFAO, la forme de la pièce de restauration dentaire voulue.

21. Procédé de fabrication d'une vitrocéramique de silicate de lithium et de quartz alpha, qui contient de 1,0 à 8,0 % en poids d'oxyde(s) d'élément(s) monovalent(s) Me^{I}₂O choisi(s) dans l'ensemble constitué par K₂O, Na₂O, Rb₂O et Cs₂O et leurs mélanges et de 1,0 à 6,0 % en poids de MgO, et qui comprend du silicate de lithium en tant que principale phase cristalline et du quartz alpha en tant qu'autre phase cristalline, dans lequel procédé
a) on soumet un verre de départ qui contient les composants de la vitrocéramique à un traitement thermique, à une température de 400 à 600 °C durant 5 à 120 minutes, pour en faire un verre de départ doté de germes,
b) et l'on soumet ce verre de départ doté de germes à un traitement thermique, à une température de 700 à 900 °C durant 5 à 120 minutes, pour en faire une vitrocéramique de silicate de lithium et de quartz alpha,
